# EUROPEAN PATENT APPLICATION

(11) **EP 1 873 528 A1**
(43) Date of publication of application: **02.01.2008**
(21) Application number: 05761911.6
(22) Date of filing: 21.06.2005
(51) Int. Cl.: G01N 33/98, A61B 5/145, A61B 5/18

(54) **DEVICES AND METHODS FOR MEASURING ETHANOL CONTENT IN BLOOD**

(30) Priority: 23.12.2004 ES 200403076
(71) Applicant: Intecsa-Inarsa S.A., E-28037 Madrid (ES)
(72) Inventor: HERNANDEZ FERNANDEZ, Julio, E-28140 Fuente el Saz (ES); MARTÍN GARZO, Luis Alfonso, E-28018 Madrid (ES); IBAÑEZ LÓPEZ, Juan Diego, E-28036 Madrid (ES); MUÑOZ PASCUAL, Francisco Javier, E-28007 Madrid (ES); RODRIGUEZ GOROSTIZA, Francisco Javier, E-08206 Sabadell (ES); REVIEJO GARCÍA, Angel Julio, E-28260 Galapagar (ES); PINGARRÓN CARRAZÓN, José Manuel, E-28020 Madrid (ES)
(74) Representative: Carpintero Lopez, Francisco
(86) International application number: PCT/ES2005/000350
(87) International publication number: WO 2006/070027

(57) **Abstract**

This invention is referred to procedures and devices constituted by graphite-teflon bienzyme amperometric biosensors for the detection of ethanol concentration in blood, directly or by means of the current measure produced by the enzymatic reactions on the electrode surface as a result of the concentration of ethanol in sweat.

## Description

One of the purposes of this invention is based on a procedure for the determination of ethanol content in blood by the measurement of the amperometrical signal obtained monitoring ethanol in sweat.

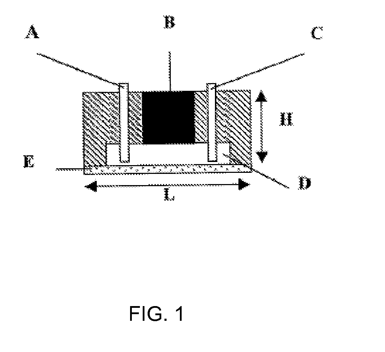

This procedure is characterized by using a device (Design 1) composed by a sensor system which includes a graphite-teflon composite bienzyme electrode as working electrode, an Ag/AgCl reference electrode and a Pt wire auxiliary electrode. These electrodes are immersed in a measuring solution separated from the skin by a membrane. The membrane needs to be not permeable to water and permeable to ethanol, as it is outlined in Figure 1.

Amperometric ethanol measures in sweat are performed using this sensor and monitoring the current change obtained by applying a fixed potential of 0.0 V versus Ag/AgCl and directly in sweat through skin. Measurements can be carried out at a pre-selected time or in a continuous way.

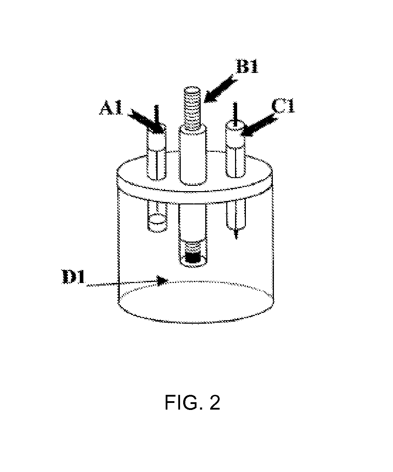

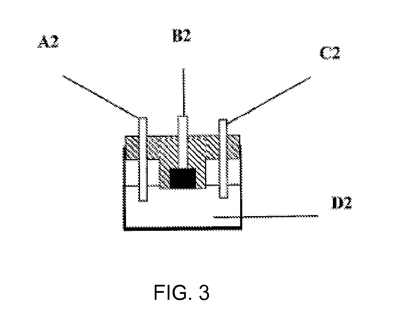

Current measured is transformed in ethanol concentration in blood employing an experimental calibration plot with a correlation coefficient of 0.92.
The second purpose of this invention is a procedure for the direct determination of ethanol in blood. This procedure is characterized by employing graphite-teflon composite bienzyme biosensors sensitive to the concentration of ethanol. This procedure can be carried out with two different devices:
Device Design 2: Electrochemical cell composed by the ethanol electrochemical biosensor and reference and auxiliary commercial electrodes immersed in the measuring solution as it can be seen in Figure 2.
Device Design 3: Electrochemical cell formed by an integrated system which includes the graphite-teflon composite bienzyme biosensor, an Ag/AgCl reference electrode and a Pt wire auxiliary electrode immersed in the measuring solution as it is shown in figure 3.

Determination of ethanol in blood can be carried out by means of two different ways: a) external calibration and b) standard addition method.

This invention is included in the measuring devices field (sensors) for the quantification of ethanol in blood by its measurement in sweet (device design 1) and directly in blood (devices design 2 and 3)

### History of the invention

In general basis, alternative equipments available in the market and designed for the measurement of ethanol in blood are not based in enzymatic reactions; these devices have bigger dimensions than those developed in this work, and also they need special installation, operating and maintenance conditions. They generate results after a comparative long of time.

The devices linked to this invention, have very small dimensions, are not very heavy and they don't require special installation, operating and maintenance conditions. They detect at low cost, concentrations of ethanol in blood in less than 10 minutes after the subject's first ingestion of ethanol, giving punctual data or high accuracy continuous results in real time.

The system is based on the use of bienzymatic processes adapted to ethanol, that do not generate any other simultaneous reaction in the sample, and on the use of a microelectronic element with a potentiostat incorporated that allows to measure in real time. Current levels are associated to the ethanol concentration present in the sample.
This way, slow and highly cost system have been changed by the devices of this invention. These devices are precise, novel and are faster in generating results.
General interest papers associated with this invention and showing in detail the technique used are divulgated in the following bibliography:
Graphite-Teflon composite bienzyme amperometric biosensors for monitoring of ethanols, A. Guzmán-Vázquez de Prada, N. Peña, M.L. Mena, A.J. Reviejo and J.M. Pingarrón, Biosensors and Bioelectronics, 18 (2003) 1279-1288.
Amperometric multidetection with composite enzyme electrodes, A. Guzmán-Vázquez de Prada, N. Peña, C.Parrado, A.J. Reviejo and J.M. Pingarrón, Talanta, A62 (2004) 896-903.

### INVENTION DESCRIPTION

The goals of this invention are the procedures and devices mentioned above for the ethanol measurement in blood, in a direct way or by means of its correlation with the signal due to the concentration of ethanol in sweat.

The devices based on the use of a graphite-teflon amperometric bienzyme biosensor for the detection of ethanol, can carry out the measure directly in blood or correlating the amperometric signal generated by the ethanol in sweat, at an applied potential of 0.0 V, with its level in blood.

The determination of ethanol in blood through its measurement in sweat can be carry out in the linear range between 0.0005 and 0.6 grams/liter. The device allows the measurement after five minutes since the first ingest.

The designed devices for the direct measurement of ethanol in blood, allows the determination in a linear range between 0.0005-0.14 grams/liter in the measuring solution.

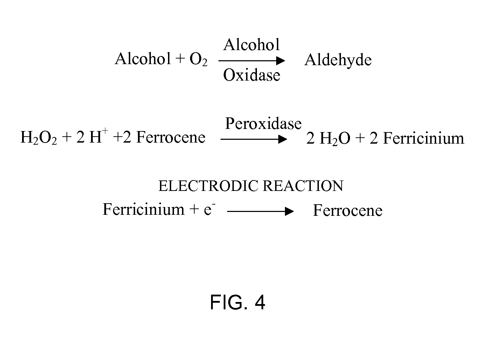

The device for the determination of ethanol in blood through the measurements of ethanol in sweat incorporates a hydrophobic permeable membrane for ethanol with a porosity between 0.30 and 6 micrometers, which allows the ethanol to pass through to the measuring solution and separate it from the skin.
The enzymatic process that allows the determination of ethanol in blood, as well as the electrodic reaction which produces the measure current, are described in Figure 4.
The microelectronic of the device permits to correlate the currents obtained on the electrodes with the concentration of ethanol in blood, through the incorporation of the calibration plot obtained experimentally.
The interest of this invention is its low cost and also its easy integration with other measurement procedures and control.
This invention is also referred to a procedure for the direct determination of ethanol in blood.

### DESCRIPTION OF THE DRAWINGS

To complete the above description and with the purpose of giving a best comprehension of its characteristics, this descriptive report includes some illustrative figures that represent the most significant details of the invention.
Figure 1. Device design 1 that includes one electrochemical cell that consists of: A Reference electrode, B Ethanol biosensor, C Auxiliary electrode, D Measuring solution containing a phosphate buffer solution with pH values between 6.0 and 8.0, E Permeable membrane for ethanol; L, length of the device between 5 and 10 mm; H height of the device between 3 and 10 mm.
Figure 2. Device design 2 that includes one electrochemical cell that consists of: A1 commercial reference electrode, B1 electrochemical biosensor of ethanol, C1 commercial auxiliary electrode immersed in D1 phosphate buffer solution with pH values between 6.0 and 8.0.
Figure 3. Device design 3 that includes one electrochemical cell that consists of: B2 graphite-teflon composite bienzyme electrode, A2 Ag/AgCl reference electrode and C2 a Pt auxiliary electrode immersed in D2 phosphate buffer solution with pH values between 6.0 and 8.0.
Figure 4. Enzymatic and electrodic reactions that occur on the devices related to this invention.

### INVENTION PREFERENTIAL EXECUTION

As it can be seen in Figure 1, a way of preferential execution of the invention can be described.
The detection procedure related to this invention implies the use of a device formed by a graphite-teflon byenzime amperometric biosensor and a membrane of appropriate dimensions in direct contact with the skin which allows ethanol presents in sweat transport through it to the measuring solution. Lately reactions described in Figure 4 are produced on the amperometric biosensor surface, originating a current intensity allowing, finally, the determination of ethanol concentration in blood.

The current generated by the enzymatic reaction at an applied potential of 0.0 V is proportional to the ethanol concentration in blood. Its quantitative determination is carried out by means of a previous experimental calibration plot which correlation coefficient is 092.
The essential of this invention is not modified by any changes in materials, shape, size and disposition of the components that have been described in no limiting way, being enough this description to be reproduced by a specialist.

## Claims

1. Device for the measurement of ethanol concentration in blood constituted by a graphite-teflon bienzyme amperometric biosensor.

2. Device for the measurement of ethanol concentration in blood according to claim 1 and **characterized** for the obtaining of the current measure produced by the enzymatic reactions on the electrode surface as a result of the concentration of ethanol in sweat.

3. Device for the measurement of ethanol in blood according to claim 1 and **characterized by** the direct obtaining of the said measurement in blood.

4. Integrated device for the measurement of ethanol in blood according to claims 1 and 2. This device incorporates an ethanol permeable and no water permeable hydrophobic membrane that separates the measuring solution from the skin.

5. Integrated device for the measurement of ethanol in blood according to claims 1 and 3 **characterized by** the direct measurement of ethanol in blood based on a drop of blood added to measuring solution.

6. Integrated device for the measurement of ethanol in blood according to claims 1, 2, 3, 4 and 5 **characterized by** including a microprocessor and a micropotentiostat allowing the correlation between the current generated on the bienzyme electrode and the ethanol concentration in blood.
